# EUROPEAN PATENT SPECIFICATION

(11) **EP 1 395 306 B1**
(45) Date of publication and mention of the grant of the patent: **31.05.2006**
(21) Application number: 02726909.1
(22) Date of filing: 21.05.2002
(51) Int. Cl.: A61L 31/02, A61L 29/02, A61B 18/14

(54) **MALLEABLE ELONGATED MEDICAL DEVICE**
VERFORMBARE LANGGESTRECKTE MEDIZINISCHE VORRICHTUNG
DISPOSITIF MEDICAL ALLONGE MALLEABLE

(30) Priority: 21.05.2001 US 292484 P
(43) Date of publication of application: 10.03.2004
(73) Proprietor: MEDTRONIC, INC., Minneapolis, Minnesota 55432-5604 (US)
(72) Inventor: SKARDA, James, R., Lake Elmo, MN 55042 (US)
(74) Representative: Hughes, Andrea Michelle
(86) International application number: PCT/US2002/016136
(87) International publication number: WO 2002/094334

(56) References cited:
- EP-A- 1 042 990
- WO-A-95/10318
- WO-A-95/19800
- WO-A-99/58184
- US-A- 4 197 643

## Description

### FIELD OF THE INVENTION

The present invention relates generally to elongated medical devices adapted to be inserted through an access pathway into a body vessel, organ or cavity to locate a therapeutic or diagnostic distal segment of the elongated medical device into alignment with an anatomic feature of interest, and particularly to such an elongated medical device having a malleable distal segment capable of being manually formed into a shape facilitating such alignment at room temperature.

### BACKGROUND OF THE INVENTION

Many elongated medical devices are known that are inserted through an access pathway into a body vessel, organ or cavity to locate a therapeutic or diagnostic distal segment of the elongated medical device into alignment with an anatomic feature of interest. For example, catheters, introducers and guide sheaths of various types, drainage tubes, and cannulas are available that extend from outside the body through an access pathway to a site of interest and provide a lumen through which fluids, materials, or other elongated medical devices are introduced to the site, or body fluids are drained or sampled from the site. Other elongated medical devices include many forms of medical electrical leads that bear sensing and/or electrical stimulation electrodes for sensing electrical signals of the body and/or applying electrical stimulation to the body, e.g. leads for pacing, cardioversion, nerve stimulation, muscle stimulation, spinal column stimulation, deep brain stimulation, etc. Other medical electrical leads bearing physiologic sensors for measuring pressure, temperature, pH, etc, in a distal segment thereof that are adapted to be placed at a site of interest are also known. Yet other elongated medical devices include guide wires that are directed through tortuous vascular pathways to locate a distal segment thereof typically within a blood vessel. A catheter, e.g. a PTCA balloon catheter for dilating constrictions in blood vessels or delivering stents and grafts, or a medical electrical lead having a through-lumen are then advanced over-the-wire to the site. Still other elongated medical devices include stiffening stylets that are placed into the lumens of medical electrical leads and in certain guide wires to impart column strength and stiffness to the assembly to enable transvenous advancement of the assembly into a heart chamber or cardiac blood vessel.

Such elongated medical devices must have flexibility to navigate the twists and turns of the access pathway, sufficient column strength in the proximal segment thereof to be pushed through the access pathway alone or over a guide wire or through a lumen, and the capability of orienting the distal segment and any electrodes or sensors or ports of the distal segment in a preferred alignment with an anatomical feature at the accessed site so that a diagnostic or therapeutic procedure can be completed. In general terms, the elongated medical device body must also resist kinking and be capable of being advanced through access pathways that twist and turn, sometimes abruptly at acute angles.

Such elongated medical devices also possess an axis of the medical device body extending between the medical device proximal and distal ends. The distal segment is typically axially aligned with the proximal segment and any intermediate segments at their junctions, either when unrestrained or when restrained over the wire or through a lumen during advancement through the access pathway.

It is commonly the practice, particularly with guide and diagnostic catheters, to provide preformed bends at the junctions between segments or pre-curved or shaped segments that are adapted to orient the distal segment and possibly intermediate segments into alignment with an anatomical feature at the accessed site. For instance, many diagnostic procedures involve placing a catheter tip into a side port across a vascular orifice to inject radiographic fluid through the catheter lumen into the vessel. Such diagnostic catheters have historically been formed of thermoplastic materials that can be heated, as in heated water, and bent into a shape that the physician can use in attempting to access the vessel opening. A considerable variety of pre-formed shapes of such catheters have been developed over the years and made available for use in such procedures. Still, the physician may find that the anatomy of any given patient may require altering the bend by heating the catheter, changing the bend and letting it cool before it is advanced to the site where it must make an abrupt change in direction.

Similarly, guide wires have been made available over the years having malleable distal tips that the physician bends to track a tortuous pathway in the vascular system. The distal end of the stylet is also typically bent or shaped by the physician to impart a curvature in the distal segment of a medical electrical lead that the stylet is inserted into in order to orient the lead distal end into alignment with an anatomical feature at the accessed site.

It is also proposed that the distal segment of a medical electrical lead be made malleable when heated to soften the thermoplastic polymer in commonly assigned U.S. Patent No. 4,154,247, particularly to facilitate making contact of an atrial pace/sense electrode with the atrial wall in the right atrium.

Other approaches have been taken to impart bends or curves in the distal segments of catheters and medical electrical leads involving use of a deflection mechanism comprising push-pull or pull wires extending between a proximal handle through the proximal segment and into a more distal segment. The deflection mechanism is manipulated to selectively deflect or straighten the distal segment and, in some cases, intermediate segments of the device body. Many versions of electrophysiology (EP) catheters have been disclosed that are designed to perform mapping and/or ablation of cardiac tissue to diagnose and treat abnormal tissue that induces or sustains cardiac arrhythmias and that employ deflectable distal and intermediate segments controlled by push-pull wire mechanisms. Highly complex shapes are sometimes found necessary to encircle a pulmonary vein orifice, for example, to ablate the left atrial wall tissue to interrupt arrhythmic pathways. For example, commonly assigned U.S. Patent Nos. 5,445,148 to Jaraczewski et al., 5,545,200 to West et al., 5,487,757 to Truckai et al., 5,823,955 to Kuck et al., and 6,002,955 to Willems et al. disclose a variety of such shapes and mechanisms for forming the shapes. The ' 148 patent discloses forming such shapes by heating and molding the thermoplastic body. The remaining patents describe various types of manipulator mechanisms for rotating and deflecting the distal section or segments thereof into complex shapes. In U.S. Patent No. 6,164,153 to Cox et al., it is suggested that a malleable metal rod be co-extruded into the distal segment of an EP mapping/ablation catheter so that it can at least be partially shaped by manipulation thereof before it is introduced to the heart, but no particular metal is identified.

Thus, the typical elongated medical device must be both flexible and kink resistant, but in certain instances must be malleable or deflectable at least in a distal segment and/or intermediate segments thereof to pre-form or alter the axial alignments of the distal and/or intermediate segments with respect to one another and the proximal segment.

The prior art makes reference to the use of alloys such as Nitinol (Ni--Ti alloy) that have shape memory and/or superelastic characteristics in elongated medical devices which are designed to be inserted into a patient's body. The "shape memory" characteristics allow the devices to be deformed to facilitate their insertion into a body lumen or cavity and then be heated within the body so that the device returns to its original shape. The "superelastic" characteristics on the other hand generally allow the alloy to be deformed and restrained in the deformed condition to facilitate the insertion of the medical device containing the alloy into a patient's body, with such deformation causing the phase transformation. Once within the body lumen, the restraint on the superelastic member can be removed, thereby reducing the stress therein so that the superelastic member can return to its original undeformed shape by the transformation back to the original phase. Alloys having shape memory/superelastic characteristics generally have at least two phases, a martensite phase, which has a relatively low tensile strength and which is stable at relatively low temperatures, and an austenite phase, which has a relatively high tensile strength and which is stable at temperatures higher than the martensite phase.

Shape memory characteristics are imparted to the alloy by heating the alloy at a temperature above which the transformation from the martensite phase to the austenite phase is complete, i.e. a temperature above which the austenite phase is stable. The shape of the alloy during this heat treatment is the shape "remembered". The heat-treated alloy is cooled to a temperature at which the martensite phase is stable, which causes the austenite phase to transform to the martensite phase. The alloy in the martensite phase is then plastically deformed, e.g. through an introducer or guide catheter lumen or over a guide wire that has been already advanced through the body passageway, or by the passageway itself, to facilitate the advancement to the site of interest. Subsequent heating of the deformed martensite phase to a temperature above the martensite to austenite transformation temperature (e.g. body temperature) causes the deformed martensite phase to transform to the austenite phase, and during this phase transformation the alloy reverts back to its original shape. Elongated catheters and guide wires employing this technique are described, for example, in U.S. Patent Nos. 3"890,977 and 5,025,799 (both to Wilson).

US 4,197,643, WO 95/19800 and WO 99/58184 all disclose medical devices comprising a Beta titanium alloy component exhibiting shape memory properties.

This approach of using the shape memory characteristics of these alloys in medical devices intended to be placed within a patient's body presented operational difficulties. For example, with Beta-titanium alloys having a stable martensite temperature below body temperature, it was frequently difficult to maintain the temperature of the medical device containing such an alloy sufficiently below body temperature to prevent the transformation of the martensite phase to the austenite phase when the device was being inserted into a patient's body. If the alloy is selected or treated to have martensite-to-austenite transformation temperature well above body temperature, the devices could be introduced into a patient's body with little or no problem. However, if the elongated medical device was made complex by the necessity of providing resistance or other heating of the shape imparting alloy element in the distal segment above body temperature. And, the martensite-to-austenite transformation temperature was frequently so high as to cause tissue damage and very high levels of pain.

When stress is applied to a specimen of an alloy such as Nitinol exhibiting superelastic characteristics at a temperature at or above which the transformation of martensite phase to the austenite phase is complete, the specimen deforms elastically until it reaches a particular stress level where the alloy then undergoes a stress-induced phase transformation from the austenite phase to the martensite phase, referred to as "stress-induced martensite". The alloy undergoes significant increases in strain but with little or no corresponding increases in stress as the phase transformation proceeds until the transformation of the austenite phase to the martensite phase is complete. Thereafter, a further increase in stress is necessary to cause further deformation. The martensitic alloy first yields elastically upon the application of additional stress and then plastically with permanent residual deformation.

The martensitic specimen will elastically recover and transform back to the austenite phase if the load on the specimen is removed before any permanent deformation has occurred. The reduction in stress first causes a decrease in strain. As stress reduction reaches the level at which the martensite phase transforms back into the austenite phase, the stress level in the specimen will remain essentially constant (but substantially less than the constant stress level at which the austenite transforms to the martensite) until the transformation back to the austenite phase is complete, i.e. there is significant recovery in strain with only negligible corresponding stress reduction. After the transformation back to austenite is complete, further stress reduction results in elastic strain reduction. This ability to incur significant strain at relatively constant stress upon the application of a load and to recover from the deformation upon the removal of the load is commonly referred to as superelasticity.

The prior art makes reference to the use of alloys having superelastic characteristics in medical devices that are to be inserted through passageways or otherwise implanted or used within a patient's body. See for example, U.S. Patent Nos. 4,665,905 (Jervis), 4,925,445 (Sakamoto et al.), 5,341,818 (Abrams et al.). In the case of elongated medical devices, e.g., catheters, guide wires, medical electrical leads, and the like, the objective of the use of a superelastic alloy element shaped as a core wire or tube or coil in the distal segment is to impart a shape memory that the distal segment assumes when unrestrained at the desired site in the body or as it is advanced in a pathway to the desired site. At the same time, the superelastic alloy element is intended to impart a great deal of flexibility to the device distal segment while maintaining column strength or pushability so that the device distal end can be directed through the twists and turns of a tortuous pathway without developing a permanent set or kink and without doubling over or inverting. The unrestrained shape is typically straight, and the proximal, distal and any intermediate segments of the device body are axially aligned. However, the unrestrained shape of a segment having a superelastic alloy element in it to impart the shape can constitute a bend or curve of the segment when unrestrained.

These characteristics of the superelastic alloys described for use with elongated medical devices are not suitable to providing a malleable distal or intermediate segment that can be shaped or plastically deformed by the physician. Consequently, where malleable distal segments for guide wires have been suggested, they involve use of core wires or coils formed of or coated with malleable metals or alloys that are placed into the segment to be pre-formed as described, for example, in U.S. Patent Nos. 5,876,356 (Viera et al), and 6,139,510 (Palermo).

However, such typical malleable metal wires or rods do not solve all the problems that are encountered in practice. It is often the case that a bend or curve that is formed by shaping the malleable segment of an elongated medical device of the types described above proves to be inadequate to access a desired site in the body, e.g., into a branching vessel or against a tissue surface. Then, it is necessary to withdraw the elongated medical device, reshape the distal segment, and to repeat the steps to see whether the new shape works better. Only limited straightening and reshaping of typical ductile alloys can be accomplished before the metal wire or rod fatigues. Moreover, it is difficult to mechanically couple typical superelastic Beta III titanium alloys with malleable metals or alloys.

A catheter guide wire using Ni--Ti--Fe alloys is described in U.S. Patent No. 5,069,226, to Yamauchi, et al. A wire formed of the Ni--Ti--Fe alloy is cold worked and at least an end section is heat-treated at a temperature of about 400° C to 500° C so that the end section exhibits pseudo-elasticity at a temperature of about 37° C (body temperature) and plasticity at a temperature below about 80° C. An orthodontic archwire is disclosed in PCT publication WO 98/02109 that is also formed of Ni--Ti--Fe alloy that is heat-treated at a temperature of about 450° C to 600° C. The archwire is characterized to be "semisuperelastic" in that it exhibits superelastic properties when subjected to strain but, when bent to an imposed strain of 8% or more, it undergoes plastic flow and resultant permanent set. An orthodontist shapes a bend in an archwire at a particular location along its length to impart force against a particular tooth to be corrected.

However, wires formed of such Ni--Ti--Fe alloys do not retain a bend or kink that is imparted to the wire when the wire is straightened out for introduction through an introducer catheter or the like. A kink imparted to Ni-Ti-Fe alloy wire that is subsequently passed through a tube so that the kink is straightened tends to not fully restore when it is released from the confines of the tube. Elongated medical devices are passed through narrow confines of body vessels and cavities such that any shape imparted to the elongated medical device is changed as it is advanced through the body pathway or a blood vessel lumen or a catheter lumen or the like. It would be difficult to employ the Ni--Ti--Fe alloy in situations where the physician carefully forms the distal segment into a desired shape but then must straighten it or allow it to be changed to another shape to enable it to be advanced to the distal site.

### SUMMARY OF THE INVENTION

The present invention is directed to an elongated medical device for introduction into a patient's body into conformance with an anatomical structure at a site of interest, comprising an elongated device body having device body proximal and distal ends and a device body axis, the device body further comprising a device body proximal section extending from the device body proximal end to a proximal section distal end and a device body distal section extending from the proximal section distal end to the device body distal end; and a malleable member extending within the device bodyalong the device body axis, characterised in that the malleable member is formed of a Beta III titanium alloy of the type exhibiting superelastic properties when subjected to strain at a bending strain of less than a set threshold strain and capable of undergoing plastic flow to be manually formed in a first set shape of a plurality of set shapes when subjected to a strain of greater than the set threshold to impart a shape enabling conformance of the distal section to the anatomical structure, the Beta III titanium alloy enabling the malleable member to be formed in a second set shape different from the first set shape during application of strain of less than the set threshold strain during introduction of the elongated medical device into a patient's body and orientation of the distal segment into conformance with the anatomical structure at the site of interest.

The malleable member is formed of a Beta III titanium alloy of the type exhibiting superelastic properties when subjected to strain at a bending strain of less than a set threshold strain and capable of undergoing plastic flow to take a set shape when subjected to a strain of greater than the set threshold at the same temperature. In this way, the distal section is malleable into a set shape by imposed bending strain exceeding the bending strain set threshold. The Beta III titanium alloy is characterized by the capability of the set shape being straightened when restrained into a relatively straight configuration and of reverting or recovering to substantially the set shape, thereby possessing shape memory of the imparted set shape. Moreover, an imparted shape, such as a bend, can be removed by shaping, e.g. bending, the malleable member in the opposite direction at a strain of greater than the set threshold at the same temperature.

The elongated medical device can include any one of a guide wire, an electrical medical lead for stimulation and/or sensing physiologic parameters, a catheter, a sheath, a cannula or any other medical tube, a stylet, or the like, in adult and pediatric sizes wherein shaping of a distal section or segment is desirable to assist in locating it at a desired site in a lumen or in anatomically conforming relation to an organ or other body tissue. Such elongated medical devices typically include a medical device body having a proximal section and a distal section that are either simply designated as such or are characterized by differing construction imparting differing handling and operating capabilities. In the latter case, the distal sections may further include proximal and distal segments and even intermediate segments each characterized by differing handling and operating capabilities. In certain elongated medical devices, steering mechanisms are employed to deflect a distal section or segment from outside the body.

In one preferred embodiment, the elongated medical device is a steerable EP mapping/ablation catheter that includes a catheter body having a proximal section and a distal section terminating in a distal end of the catheter body. A handle is coupled to the proximal end of the catheter body, and manipulators enable the deflection of at least a distal segment of the distal section with respect to a proximal segment of the distal section or the proximal section. At least one distal tip electrode is preferably confined to the distal segment that can have a straight distal segment axis or can have a pre-formed curvature of the distal segment axis extending distally from the intermediate segment. An elongated malleable member is formed that extends through at least one of the distal segment, the proximal segment or both. The elongated malleable member can also extend between the proximal end and the distal end of the catheter body. The malleable member is preferably co-extruded into at least the distal section of the catheter body that it extends through, and is preferably also fixed at its proximal end to the catheter body or to the handle, if the malleable member extends to the handle, and at its distal end to the catheter body or the catheter distal end. The distal section of the catheter body is formed of a relatively flexible material having flexible conductors and manipulator or push-pull wires extending through it that enables the distal section and, optionally, segments thereof, to be formed into shapes curves, bends and coils. In accordance with this aspect of the invention, the malleable member and the segment or section that it is contained within can be manually shaped to assume a desired set shape prior to implantation and can assume that set shape in relation to the anatomy of the heart wall and/or coronary vessels subject to force exerted against it by the anatomy. Moreover, the manipulator can be employed to selectively alter the shape in situ to enhance the conformance with the anatomy. Other types of catheters, electrical medical leads, cannulas, tubes, etc., having a manipulator for selectively deflecting the distal section can be formed in a similar manner.

Other catheters, electrical medical leads, guide wires, cannulas, tubes, etc., that do not employ a deflectable tip mechanism can employ the malleable member in a distal section, one or more segment of a distal section or extending the full length of the proximal and distal sections. Typically the distal segment(s) or section includes at least one malleable member within or on the device body that can be manipulated to form the set shape.

Advantageously, an imparted set shape does not affect the superelastic characteristics of the malleable member so that it may be restrained or straightened during the implantation procedure to the anatomical site by applying a strain less than the set threshold strain yet possesses nearly full recovery of the set shape when no longer restrained at the site. Moreover, the malleable member can again be manipulated by subsequently applying a strain exceeding the set threshold strain against a set shape to reverse the set shape or to impart a new set shape.

### BRIEF DESCRIPTION OF THE DRAWINGS

These and other features and advantages of the invention will become apparent from the following description in which the preferred embodiments are disclosed in detail (by way of example only) in conjunction with the accompanying drawings in which:
FIG. 1 is an overall view of one embodiment of an EP mapping and/or ablation (mapping/ablation) catheter made according to the invention that can be shaped into a variety of configurations;
FIGs. 2A-2H are simplified views of the distal section of the catheter body of FIG. 1 showing various types of shapes that can be imparted by forming bends in the malleable member traversing the distal section or segments of the distal section of the EP mapping/ablation catheter of the present invention;
FIGs. 3-8 are end cross-section views along section 3,8 - 3, 8 of FIG. 1 illustrating various alternative forms of malleable members extending through the distal section of the EP mapping/ablation catheter body;
FIG. 9 is an overall view of one generic catheter made according to the invention incorporating a malleable member whereby the distal section or a segment thereof can be shaped into a variety of configurations as illustrated in FIGs. 2A-2H, for example;
FIGs. 10-11 are end cross-section views along section 10, 11 - 10, 11 of FIG. 9 illustrating various alternative forms of malleable members extending through the distal section of the catheter body;
FIG. 12 is an overall view of one generic guide wire made according to the invention incorporating a malleable member whereby the distal section or a segment thereof can be shaped into a variety of configurations as illustrated in FIGs. 2A-2H, for example;
FIG. 13, is a side cross-section view along section 13-13 of FIG. 12 illustrating a malleable member traversing the distal section of the guide wire body;
FIG. 14 is an overall view of one medical electrical lead made according to the invention incorporating a malleable member whereby the distal section or a segment thereof can be shaped into a variety of configurations as illustrated in FIGs. 2A-2H, for example; and
FIG. 15-16 are end cross-section views along section 15, 16 - 15, 16 of FIG. 14 illustrating various alternative forms of malleable members extending through the distal section of the lead body;

### DETAILED DESCRIPTION OF THE PREFERRED EMBODIMENTS

The present invention may be implemented in a wide variety of elongated medical devices to facilitate advancement of the device distal end or distal section through constricted and twisting access pathways, including the vascular system, of the body and/or to alignment of the distal section or segments thereof into conformance with an anatomical structure at a site of interest. Four exemplary embodiments are described in further detail.

The malleable member employed in at least the distal section of an elongated medical device body of the present invention includes a Beta III titanium alloy of the types recognized in the industry. For example, Beta III titanium alloys are characterized and classified in the paper entitled "Overview: Microstructure and Properties of Beta Titanium Alloys", by T. W. Duerig et al., Proc. Conference: Beta Titanium Alloys in the 1980's pp. 19-67, Atlanta, GA, March 8, 1983, published by The Metallurgical Society/AIME. The compositions of commercially available Beta III titanium alloys are known.

### EP Mapping/Ablation Catheter Embodiment:

The heart includes a number of pathways through which electrical signals necessary for normal, electrical and mechanical synchronous function of the upper and lower heart chambers propagate. Tachycardia, that is abnormally rapid rhythms of the heart, are caused by the presence of an arrhythmogenic site or accessory pathway which bypasses or short circuits the nodal pathways in the heart. Tachycardias may be categorized as ventricular tachycardias (VTs) or supraventricular tachycardias (SVTs). The most common SVTs include atrioventricular nodal reentrant tachycardia (AVNRT), Atrioventricular reentrant tachycardia (AVRT), atrial fibrillation (AF), and atrial flutter (AFI). Reentrant tachycardias originate in the atria and are typically caused by an accessory pathway or inappropriate premature return excitation from the ventricle through the AV node or left sided accessory pathway. Conditions such as AF and AF1 involve either premature excitation from focal ectopic sites within the atria or excitations coming through inter-atrial reentry pathways as well as regions of slow conduction within the atria. VT's originate from within the ventricles and have their entire circuit contained within the ventricles. These VT's include bundle branch reentrant tachycardia (BBR), right ventricular outflow tract tachycardia (RVOT), and ventricular fibrillation (VF). VT's are often caused by arrhythmogenic sites associated with a prior myocardial infarction as well as reentrant pathways between the ventricles. BBR involves an inappropriate conduction circuit that uses the right and left bundle branches. RVOT can be described as a tachycardia originating from the right ventricular outflow tract which involves ectopic triggering or reentry mechanisms. VF is a life threatening condition where the ventricles entertain a continuous uncoordinated series of contractions that cause a cessation of blood flow from the heart. If normal sinus rhythm is not restored, the condition is terminal.

Treatment of both SVTs and VTs may be accomplished by a variety of approaches, including drugs, surgery, implantable electrical stimulators, and catheter ablation of cardiac tissue of an affected pathway. While drugs may be the treatment of choice for many patients, drugs typically only mask the symptoms and do not cure the underlying cause. Implantable electrical stimulators, e.g., pacemakers, afferent nerve stimulators and cardioverter/defibrillators, which have proven to be a very successful treatment, usually can only correct an arrhythmia after it occurs and is successfully detected. Surgical and catheter-based treatments, in contrast, will actually cure the problem usually by ablating the abnormal arrhythmogenic tissue or accessory pathway responsible for the tachycardia. The catheter-based treatments rely on the application of various destructive energy sources to the target tissue including direct current electrical energy, radio frequency (RF) electrical energy, laser energy, ultrasound, microwaves, and the like.

RF ablation protocols have proven to be highly effective in treatment of many cardiac arrhythmias while exposing the patient to minimum side effects and risks. RF catheter ablation is generally performed after an initial electrophysiologic (EP) mapping procedure is conducted using an EP mapping catheter to locate the arrhythmogenic sites and accessory pathways. After EP mapping, an RF ablation catheter having a suitable electrode(s) is introduced to the appropriate heart chamber and manipulated so that the electrode(s) lies proximate the target tissue. Such catheters designed for mapping and ablation, frequently include one or more cylindrical or band-shaped individual electrodes mounted to the distal section of the catheter so as to facilitate mapping of a wider area in less time, or to improve access to target sites for ablation. RF energy is then applied through the electrode(s) to the cardiac tissue to ablate a region of the tissue that forms part of the arrhythmogenic site or the accessory pathway.

Such mapping and ablation catheters are inserted into a major vein or artery, usually in the neck or groin area, and guided into the chambers of the heart by appropriate manipulation through a venous or arterial route, respectively. The catheter must have a great deal of flexibility or steerability to be advanced through the vascular system into a chamber of the heart, and the catheter must permit user manipulation of the tip even when the catheter body traverses a curved and twisted vascular access pathway. Such catheters must facilitate manipulation of the distal tip so that the distal electrode(s) can be positioned and held against the tissue region to be mapped or ablated.

The arrhythmogenic sites or accessory pathways to be mapped and ablated frequently occur within the left atrial wall, particularly around pulmonary vein orifices. It is preferable in such cases to introduce an instrument into the right atrium by a venous route including the inferior vena cava and to advance it through the septum separating the right and left atrium (although other routes include trans-thoracically through the pericardium and onto the epicardial surface or through an incision in the left atrial wall that accesses left atrial chamber in which the present invention could be used). Typically, a guide catheter is inserted in this manner into the right atrium, and instruments are introduced through the guide catheter lumen that are manipulated from their proximal end and advanced through the septal wall first creating a very small trans-septal perforation, and then enlarging the perforation by dilation or the like. Then, the guide catheter is then advanced over the instruments through the septal wall to locate the guide catheter distal end within the left atrial chamber, and the penetrating instruments are retracted from the guide catheter lumen. The proximal end of the guide catheter is typically taped to the patient's body or a support to inhibit retraction back into the right atrial chamber. The mapping and ablation catheters are then inserted through the guide catheter lumen to locate their distal segments within the left atrial chamber. The mapping and ablation procedures are undertaken, the mapping and ablation catheters are retracted, and the guide catheter is also retracted. The trans-septal perforation tends to shrink as the dilated myocardial tissue expands across the perforation.

EP mapping/ablation catheters are typically formed with an elongated catheter body having proximal and distal catheter body ends and a catheter body axis, a handle coupled to the catheter body proximal end, a catheter body proximal section joined at a proximal section distal end with a catheter body distal section. The distal section or a distal segment thereof supports at least one electrode but typically a plurality of electrodes for sensing cardiac signals during mapping and for delivering ablation energy during ablation. An electrical conductor extends through the catheter body from each such electrode to the handle that is adapted to be coupled to a signal amplifier and/or ablation energy generator.

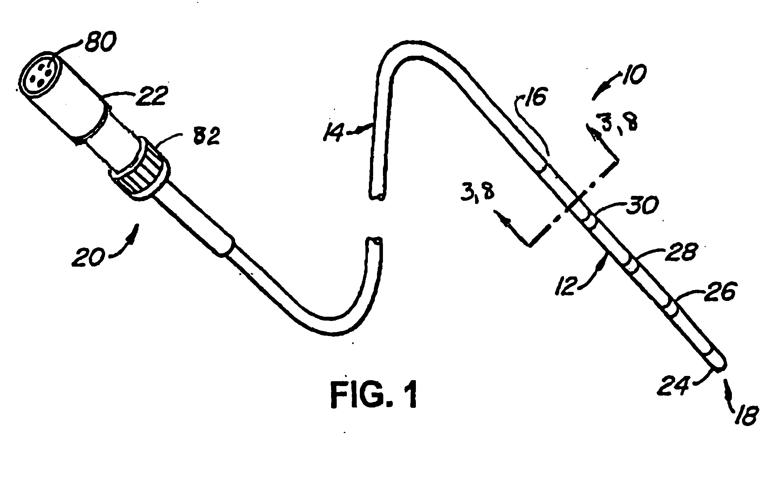

Referring to FIG. 1, an EP mapping/ablation catheter 10 constructed in accordance with the principles of the present invention includes a flexible distal section 12 joined at an attachment junction 16, usually by thermal welding, to a proximal shaft section 14. The EP mapping/ablation catheter 10 has catheter body extending between a catheter body distal end 18 and a catheter body proximal end 20. The catheter body has an overall length typically in the range from about 60 cm to 150 cm with lengths of 80 cm and 125 cm being usual for subclavian and femoral entry, respectively. The flexible distal section 12 typically has a length in the range from about 1 cm to 20 cm, usually being about 5 cm, with the remaining length of the catheter being in the proximal section 14. The distal section 12 can include two or more segments having differing characteristics, and in that case, the distal section 12 can be longer than 10 cm. A proximal handle 22 is secured to the proximal end 20 of the EP mapping/ablation catheter 10.

A plurality of electrodes are arrayed along the distal section 12 of the EP mapping/ablation catheter 10 in order to permit ECG mapping and/or ablation in the manner described above. The electrodes typically include a tip electrode 24 or an electrode spaced closely spaced from the catheter body distal end 18 and at least one proximally spaced-apart band electrode 26. Usually, at least two additional band electrodes 28 and 30 are provided, and the catheter 10 may include up to a total of ten or more electrodes. The spacing between electrodes is not critical, with adjacent electrodes usually being spaced from 2 min to 1 cm apart, typically being about 5 mm apart. The spacing between adjacent electrodes may be the same or different, with a variety of particular spacing patterns being known in the art. Each electrode is coupled through a conductor within the catheter body to connector terminals of the handle 22. The electrodes 24, 26, 28, and 30 are preferably composed of a platinum-iridium alloy. A thermocouple is also typically included in the distal segment 12, and separately insulated thermocouple conductors extend proximally through the catheter body to terminals of the cable connector 80 that are coupled via a cable to the temperature display and ablation energy control apparatus known in the art.

The handle 22 includes an elongated housing that is attached to the catheter body proximal end 20 and can be grasped by the physician during introduction of the catheter body and manipulation of the distal section 12. A cable connector 80 at the proximal end of the handle housing permits connection of the EP mapping/ablation catheter 10 to standard EKG recording and interpretation equipment and the ATAKR™ II RF Power Generator Model No. 4803 commercially available from MEDTRONIC, INC., Minneapolis, MN, for example, and others. The cable connector 80 includes a number of connector pins corresponding to the number of electrodes 24, 26, 28, 30 to permit proper connection. Use of the EP mapping/ablation catheter 10 and ECG mapping and/or ablation can be performed in a generally conventional manner as described above.

The present invention can be implemented in such an EP mapping/ablation catheter 10 having manipulators for deflecting the distal section 12 or one or more segment thereof of the types referred to above or in an EP mapping/ablation catheter without any manipulator or remote deflection capability. In the depicted EP mapping/ablation catheter 10, a single manipulator ring 82 is depicted which is coupled to a push-pull or pull wire extending through a lumen within the catheter body to a coupling at the catheter body distal tip 18 that enables the deflection of the distal section 12 from its pre-formed shape created in accordance with the present invention into a deflected configuration in at least one plane, for example. The general construction and use of such manipulators, including two or more manipulator rings that can be moved axially along or rotated about the housing of the handle 22 and associated push-pull or torque wires coupled distally to separate segments of the distal section for imparting deflections in multiple planes are disclosed in the above-referenced '200, '757, and Willems and Kuck '955 patents, for example, and they could be employed in the present invention.

The proximal shaft section 14 of EP mapping/ablation catheter 10 includes a polymeric tube having a central lumen that is coaxial with the lumen of the flexible distal section 12, although its cross-section can differ. The polymeric tube in the proximal section 14 is composed of a thermoplastic polymer and is reinforced with a braided layer, typically a stainless steel braid embedded within the polymer. A polymer, e.g., nylon, polyether block copolymers (e.g., PEBAX®, Atochem, Germany), polyolefins (e.g., Hytrel®, DuPont, Wilmington, Del.), and the like, can be employed, although a polyurethane, e.g., Pellethane 2363, typically having a hardness in the range from about 35 D to 75D, is preferred. The polymer hardness and the braid characteristics, such as pick, angle, spacing, the nature of the strand (i.e. flat or round), and the like, can be selected to provide a desired torsional stiffness and axial flexibility of the proximal shaft section 14. In an exemplary embodiment, the braid is 304 LV stainless steel formed from 0.003 in. diameter round strands at a 60° - 65° braid angle. Usually, the composite of the thermoplastic tube and braided layer will be fabricated by placing a first tube over the braided layer and a second tube within the lumen of the braided layer, and then heating the composite structure so that the thermoplastic material impregnates the braid to form a unitary structure. Alternatively, the proximal shaft section 14 is formed in a continuous process where the thermoplastic polymer is continuously extruded over the braided layer. The proximal section 14 has sufficient column strength and is capable of good torque transmission to permit controlled placement of the distal section 12 at a target site in or on the heart.

In accordance with the present invention, the distal section 12 or one or more segments thereof can be manually shaped into a non-straight configuration when not restrained during introduction and advancement through a body passageway or by use of any manipulator that facilitates such introduction and advancement or the alignment of the electrodes in a pre-selected conformation with the anatomical structure of interest, in this case against the endocardial or epicardial heart wall. This manual shaping capacity is effected by forming the distal section 12 of a flexible polymeric material that can be deflected by retraction of push-pull wire(s) and/or by an elongated, malleable member disposed within the device body extending in the direction of the device body axis.

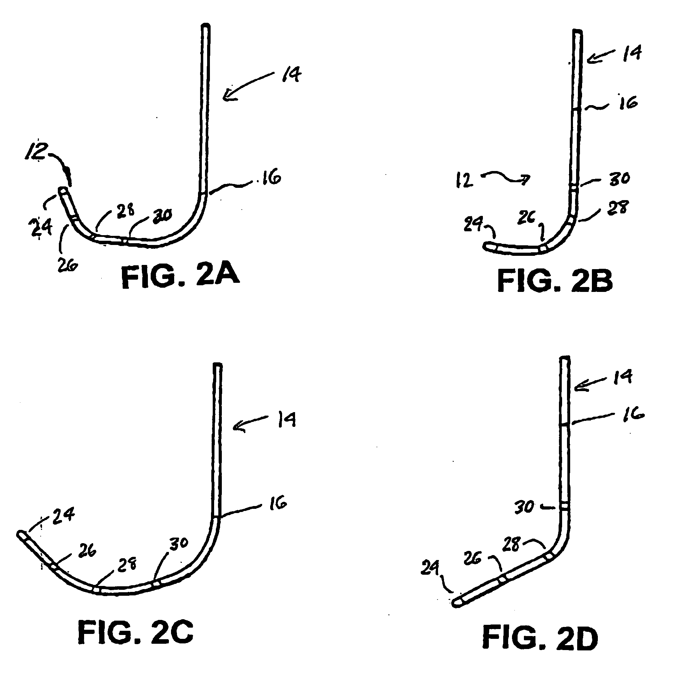

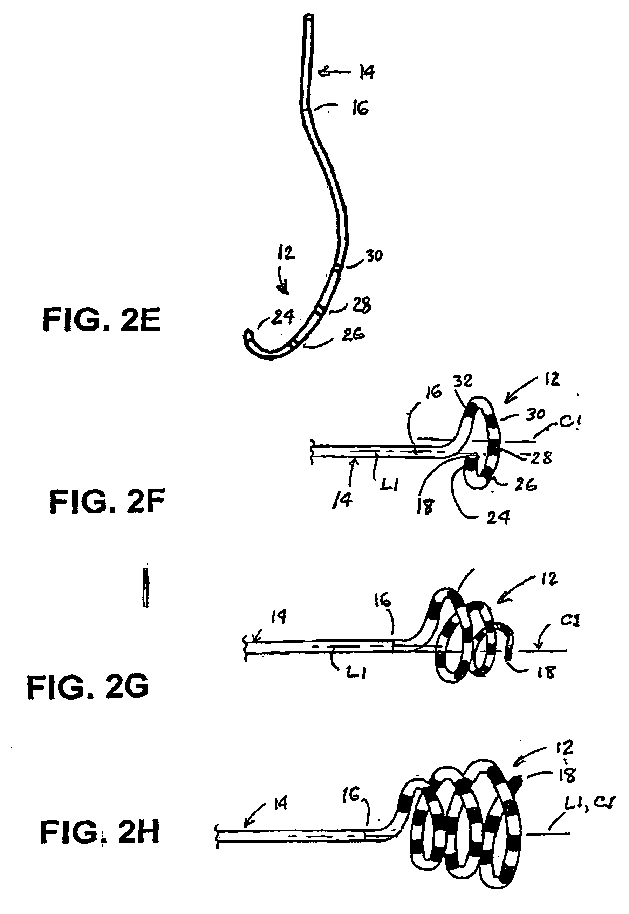

A number of exemplary formed shapes of the catheter body distal section 12 effected by bending the internally disposed malleable member into one or more, generally single plane, set shape along its length in the distal section 12 are depicted in FIGS. 2A-2E. The configuration of FIG. 2A is useful for mapping the HIS bundle; the configuration of FIG. 2B is useful for mapping according to the Josephson procedure; the configuration of FIG. 2C is useful for mapping the coronary sinus; the configuration of FIG. 2D is useful for performing conduction studies; and the configuration of FIG. 2E is useful to hook the coronary sinus (CS) from a superior approach to conduct EP studies mapping EGMs from the left side of the heart without actually entering a left heart chamber. A serpentine, single plane, set shape that can be formed in the distal section of the EP mapping/ablation catheter 10 is disclosed, for example, in U.S. Patent No. 5,782,828 to Chen et al.

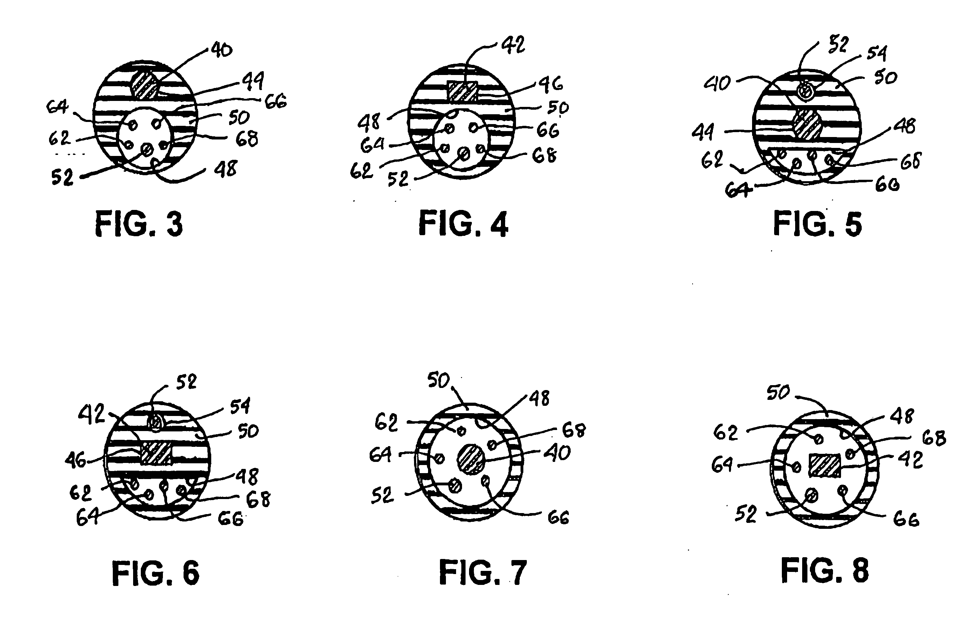

Further idealized coil or spiral shapes are depicted in FIGs. 2F-2H that are useful in mapping and ablating tissue around the ostium of a coronary vessel, particularly for ablating atrial myocardial tissue in the atrial wall surrounding the ostium of a pulmonary vein as described in commonly assigned U.S. Patent No. 6,325,797 filed April 5, 1999, for ABLATION CATHETER AND METHOD FOR ISOLATING A PULMONARY VEIN. In this case, the catheter distal section 12 can include additional ring electrodes such as ring electrode 32 of FIG. 2F so as to contact the atrial wall tissue surrounding the ostium. The coils can be formed by making successive bends in segments of the malleable member 40, 42 (FIG. 3) between the ring electrodes to approximate these idealized shapes. Certain of the coils are formed with coil axis C1 that are aligned with the catheter body axis L1 extending from the relatively straight proximal section 14, and other coils can be formed with the coil axis C1 offset from the catheter body axis L1.

Alternative cross-section configurations of the distal section are depicted in FIGs. 3-8 depicting a circular cross-section malleable member 40 or a rectangular cross-section malleable member 42 either co-extruded with or disposed within a separately extruded lumen 44, 46 or 48 of the distal catheter body 50. The cross-section views also illustrate, in this example, four electrically insulated lead conductors 62, 64, 66, 68 extending through the lumen 48 that are coupled to pins of the proximal connector 80 and the electrodes 24, 26, 28 and 30. A push-pull or pull wire 52 extends through the common lumen 48 or a separate lumen 54 between a distal attachment point, typically near the catheter body distal end 18 and the ring 82 on handle 22. It will be understood that the proximal shaft section 14 can be formed having a single lumen that the push-pull or pull wire 52, the insulated lead conductors 62, 64, 66, and 68 and, optionally, the malleable member 40, 42 extend through to the handle 22.

The distal catheter body 50 is preferably extruded of a low durometer, thermoplastic material, typically a polyurethane having a durometer in the range from 30A to 75D, preferably 55D. A suitable polymeric material for the flexible distal section 12 is Pellethane available from Dow Chemical Co., Midland, MI.. Other suitable materials for the polymeric tube include nylon, polyether block copolymers (e.g., PEBAX®, Atochem, Germany), polyolefins (e.g., Hytrel®, DuPont, Wilmington, Del.), and the like. The relatively soft, un-reinforced catheter body and the enclosed conductors and push-pull or pull wire can be readily bent by manipulation of the malleable member 40 or 42 and deflected by the manipulator comprising the push-pull or pull wire 52 and manipulator ring 82. By contrast, the relatively stiffer, proximal shaft section 14 is not amenable to and resists being manually shaped, bent or deflected in these ways.

The malleable member 40 or 42 can extend through the distal section 12 or a segment thereof and proximally to the junction 16 or all the way to the handle 22. the proximal shaft section 14 is attached directly to the proximal end of the flexible distal section 12, typically by heat welding. Preferably, the malleable member distal end is locked to the distal tip 18, and the malleable member proximal end is either locked to the handle 22 or to a a hard plastic member formed of a relatively rigid PEEK (polyether-etherketone) or other hard, temperature-resistant material at or near the junction 16 to lessen the tendency of the malleable member to twist or rotate when a bend is formed in it. The co-extrusion of the malleable member with the catheter body 50 also lessens the tendency of the malleable member to twist or rotated as the distal section or a segment thereof is manipulated to form a shape of the types illustrated in FIGS. 2A-2H, for example.

The malleable member 40 or 42 is formed of a Beta III titanium alloy of the type exhibiting superelastic properties when subjected to strain at a bending strain of less than a set threshold strain and capable of undergoing plastic flow to take a set shape when subjected to a strain of greater than the set threshold at the same temperature, that is room temperature in this case. In accordance with the invention, the set shape is reversible so that any manually formed set shape of the malleable member that does not work in enabling conformance of the distal section to the anatomical structure of interest can be reversed. Moreover, the Beta III titanium alloy enables the set shape of the malleable member 40, 42 to be restored to substantially the imparted set shape following application of strain of less than the set threshold strain during introduction of the elongated EP mapping/ablation catheter 10 into a patient's body and orientation of the distal electrodes 24, 26, 28, 30 into conformance with the anatomical structure at the site of interest.

Other modification and variation can be made to the disclosed embodiments without departing from the subject of the invention as defined in the following claims. For example, materials, diameters and lengths can be changed to suit the particular needs or desires of the user. A single mapping/ablation electrode, or more than two mapping/ablation electrodes could be present. A plurality of small sized mapping electrodes displaced apart along the distal section of the catheter body are typically provided and paired electrically to increase sensing resolution of the electrical signals of the heart traversing the adjoining heart wall site. Mapping electrodes could also be located between ablation electrodes. In some cases it may be desired to apply energy to more than one ablation electrode at the same time; for example, four ablation electrodes could be used and powered in pairs. Other modifications will occur to those of skill in the art.

### Catheter Embodiment:

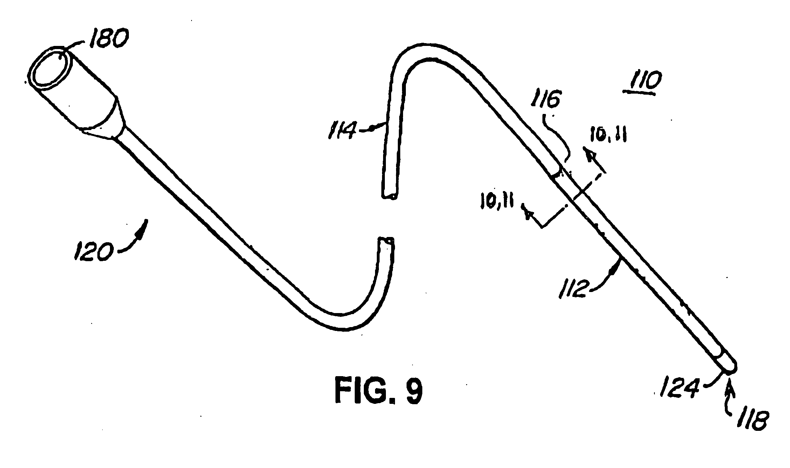

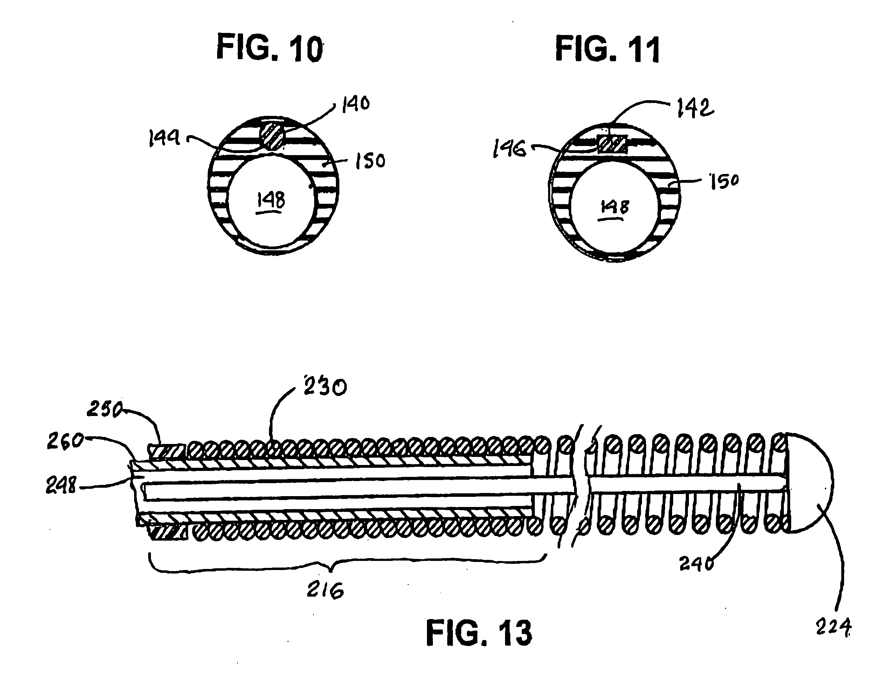

The catheters of the present invention can include relatively simple introducers, sheaths, cannulas, urologic catheters, drainage catheters and tubes, and the like, as well as more complex, coronary sinus (CS) catheters, angiography catheters, catheters for locating pulmonary veins, intra-cardiac echo (ICE) catheters, aortic bypass catheters, PTCA and stent delivery balloon catheters, other balloon catheters, and the like (hereafter referred to generically as "catheters"), in a wide variety of lengths and diameters. FIGs. 9-11 illustrate a generic catheter 110 made according to the invention incorporating a malleable member 140 or 142 whereby the distal section 112 or a segment thereof can be shaped into a variety of configurations as illustrated in FIGs. 2A-2H, for example. Other set shapes that can be formed in the distal section 112 of the catheter 110 are disclosed, for example, in the above-referenced '828 patent and in U.S. Patent Nos. 5,299,574 to Bower and 5,306,263 to Voda et al. for coronary arteriography procedures.

The catheter body extends from a proximal fitting or coupling 180 that may take any of the conventional configurations, including side ports, penetrable valves, or the like, to a catheter body distal end 118 and encloses at least one lumen 148 extending between proximal and distal lumen end openings. Preferably, at least one radiopaque ring 124 is incorporated at the distal end 118 to enable remote visualization of its location in the body.

The proximal catheter body shaft section 114 can be formed of a thermoplastic polymer incorporating a reinforcing structure, e.g., the wire braid as described above or hypotube or coiled wire, or the like, to provide steerability and pushability of the catheter 110 by itself or over a guide wire or other elongated medical device already introduced through an access pathway in the body. The distal shaft section 112 can also be formed as described above without a reinforcing structure, although a flexible wire coil can be incorporated into the sidewall. The proximal and distal shaft sections 114 and 112 are joined at a junction 116 by conventional processes.

The malleable member 140 or 142 can extend through the distal section 112 or a segment thereof and proximally to the junction 116 or all the way to the proximal coupling 180. Preferably, the malleable member distal end is locked to the distal tip 118, and the malleable member proximal end is either locked to the coupling 180 or to a hard plastic member formed of a relatively rigid PEEK or other hard material at or near the junction 116 to lessen the tendency of the malleable member to twist or rotate when a bend is formed in it. The co-extrusion of the malleable member with the catheter body 150 also lessens the tendency of the malleable member to twist or rotated as the distal section or a segment thereof is manipulated to form a shape of the types illustrated in FIGS. 2A-2H, for example.

The malleable member 140 or 142 is formed of a Beta III titanium alloy of the type exhibiting superelastic properties when subjected to strain at a bending strain of less than a set threshold strain and capable of undergoing plastic flow to take a set shape when subjected to a strain of greater than the set threshold at the same temperature, that is room temperature in this case. In accordance with the invention, the set shape is reversible so that any manually formed set shape of the malleable member that does not work in enabling conformance of the distal section to the anatomical structure of interest can be reversed. Moreover, the Beta III titanium alloy enables the set shape of the malleable member 140, 142 to be restored to substantially the imparted set shape following application of strain of less than the set threshold strain during introduction of the catheter 110 into a patient's body.

This basic structure and technique of incorporating the malleable member 140 or 142 into a catheter 110 can be followed in more complex catheters having multiple coaxial or parallel lumens, distal inflatable balloons, side ports, and the like. In certain cases, it may be desirable to extend the malleable member 140 or 142 through a common lumen 148 as in FIGs. 7 and 8, particularly if the catheter distal tip 118 is distal to an inflation balloon or other structure. Other modifications will occur to those of skill in the art.

### Guide Wire Embodiment:

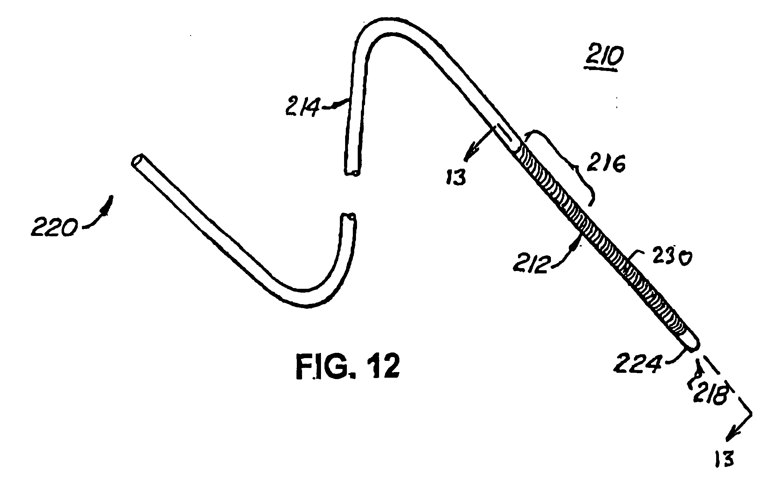

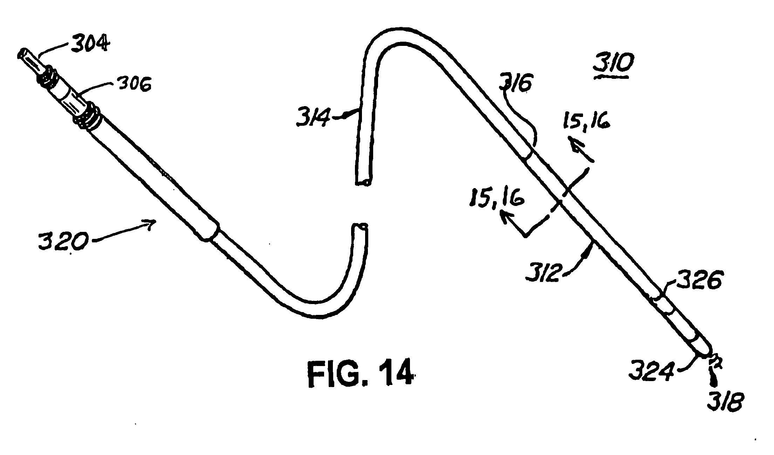

The present invention can be implemented in other elongated medical guide wires, including guide wires of the type including stylets, infusion wires, balloon inflation guide wires, and the like, with a fixed core wire or with a removable core wire (hereafter referred to generically as "guide wires") in a wide variety of lengths and diameters. FIGs. 12 and 13 illustrate such a generic guide wire 210 made according to the invention incorporating a malleable member 240 whereby the distal section 212 or a segment thereof can be shaped into a variety of configurations as illustrated in FIGs. 2A-2H, for example. The guide wire body extends from a proximal end 220 that can take any of the conventional configurations, including fluid ports or the like, to guide wire body distal end 218 and encloses at least one lumen 248 extending between the proximal end 220 and a radiopaque distal tip plug 224.

The proximal catheter body shaft section 214 can be formed of a thermoplastic polymer tube 250 overlying or incorporating a reinforcing structure, e.g., the wire braid as described above or a coiled wire, or the like, or the depicted hypotube 260 to provide steerability and pushability of the guide wire 210 by itself or through the lumen of a catheter or other elongated medical device already introduced through an access pathway in the body. The distal shaft section 212 is preferably formed with a flexible wire coil 230 extending distally over a distal extension of the hypotube 260. The coil 230 is close wound where it is supported over the distal extension of the hypotube 260 and more loosely wound in the unsupported distal section extending to the distal tip plug 224. The proximal and distal shaft sections 214 and 212 are joined at the elongated junction 216 by conventional processes including welding or adhesives. The distal tip plug 224 is preferably welded to the distal end of the wire coil 230. The lumen 248 can be employed to deliver infusates or to sample body fluids and pressures in a manner well known in the art if a suitable lumen opening is provided at the guide wire proximal end 220.

The malleable member 240 can be circular or rectangular in cross-section and can extend through the distal section 212 proximally to the junction 216 where its proximal end can be attached to the hypotube 260 or can extend all the way to the guide wire proximal end 220. Preferably, the malleable member distal end is locked into or crimped to the distal tip plug 224, and the malleable member proximal end is locked or crimped to the proximal end 220 to lessen the tendency of the malleable member to twist or rotate when the distal section or a segment thereof is manipulated to form a shape of the types illustrated in FIGS. 2A-2H, for example.

The malleable member 240 is formed of a Beta III titanium alloy of the type exhibiting superelastic properties when subjected to strain at a bending strain of less than a set threshold strain and capable of undergoing plastic flow to take a set shape when subjected to a strain of greater than the set threshold at the same temperature, that is room temperature in this case. In accordance with the invention, the set shape is reversible so that any manually formed set shape of the malleable member that does not work in enabling conformance of the distal section to the anatomical structure of interest can be reversed. Moreover, the Beta III titanium alloy enables the set shape of the malleable member 240 to be restored to substantially the imparted set shape following application of strain of less than the set threshold strain during introduction of the guide wire 210 into a patient's body.

The malleable member distal section can be tapered distally to make it the distal section 212 more flexible distally. The malleable member 240 can completely fill guide wire lumen 248, and the hypotube 260 or other reinforcement of the proximal section can also be eliminated. The malleable member 240 can also constitute a removable core wire that can be inserted into or withdrawn from the guide wire 248 to stiffen or shape it during introduction and to provide a fluid delivery or withdrawal lumen 248 for infusion or pressure measurement as is well known in the art. Other modifications will occur to those of skill in the art.

### Medical Electrical Lead Embodiment:

The present invention can be implemented in other elongated medical electrical leads, including leads of the type that bear sensing and/or electrical stimulation electrodes for sensing electrical signals of the body and/or applying electrical stimulation to the body, e.g. leads for pacing, cardioversion, nerve stimulation, muscle stimulation, spinal column stimulation, deep brain stimulation, etc., as well as medical electrical leads bearing physiologic sensors for measuring pressure, temperature, pH, etc, in a distal segment thereof that are adapted to be placed at a site of interest. All such medical electrical leads include electrical sensing and/or stimulation leads and/or physiologic sensors, electrical conductors encased within the lead body and proximal connector elements enabling connection with an implantable pulse generator and/or monitor.

FIGs. 14 - 16 illustrate such a generic medical electrical lead 310 made according to the invention incorporating a malleable member 340 whereby the distal section 312 or a segment thereof can be shaped into a variety of configurations as illustrated in FIGs. 2A-2H, for example. The lead body extends from a proximal end 320 that can take any of the conventional configurations, including the depicted in-line connector 380 or a bifurcated connector assembly, and extends to the lead body distal end 318.

In this example, an endocardial pacing lead 320 is illustrated having at least two pace/sense electrodes 324 and 326 located in the distal section 312. In this example, the distal end can include a distal fixation mechanism e.g., an extendable/retractable screw that is screwed into the myocardium to actively fix the distal electrode 324 (and may constitute the distal electrode), a set of flexible pliant tines that engage trabeculae to passively affix the distal electrode 324, or a tapered nonconductive extension that facilitates retention and placement of a small diameter lead body into a coronary blood vessel, e.g., the coronary sinus, all of which are well known in the art. The illustrated endocardial pacing lead 320 can be employed to perform pacing in one of the right atrium or right ventricle. The medical electrical lead could include an elongated cardioversion/defibrillation electrode, one or more proximally located pace/sense electrodes, and/or a physiologic sensor for measuring blood pressure, temperature, pH, etc.

The lead body 350 can be fabricated in a variety of ways and preferably encloses at least one lumen (in this example several side-by-side lumens) extending between the lead body proximal end 320 and distal end 318 or terminating more proximally at a sensor or electrode. The lead body 350 also encases electrically insulated conductors extending between the distal tip and ring electrodes 324 and 326 and proximal connector rings 304 and 306, respectively. Typically, the catheter body 350 is relatively uniform in construction throughout its length except that the segment between the proximal pace/sense electrode 326 and the distal pace/sense electrode 324 includes only a single electrical conductor and can be more flexible than the lead body proximal to the pace/sense electrode 324.

The designation of the proximal section 314 and the distal section 312 that can be shaped by manipulation of the malleable member is therefore somewhat arbitrary, but is generally related to the point where the lead body extends from a heart chamber (typically the right atrium) or a coronary vessel (e.g., the coronary sinus ostium). It may be advantageous to form a bend or other shape in various segments of the arbitrarily designated distal section 312 so as to conform one segment to the atrial wall or another structure of a heart chamber and direct another segment into the right ventricle or through a septum or into a coronary vessel. A number of such potential shapes are depicted in the above-referenced commonly assigned '247 patent. A similar process can be employed in the use of such medical electrical leads in other body cavities, chambers, vessels or organs.

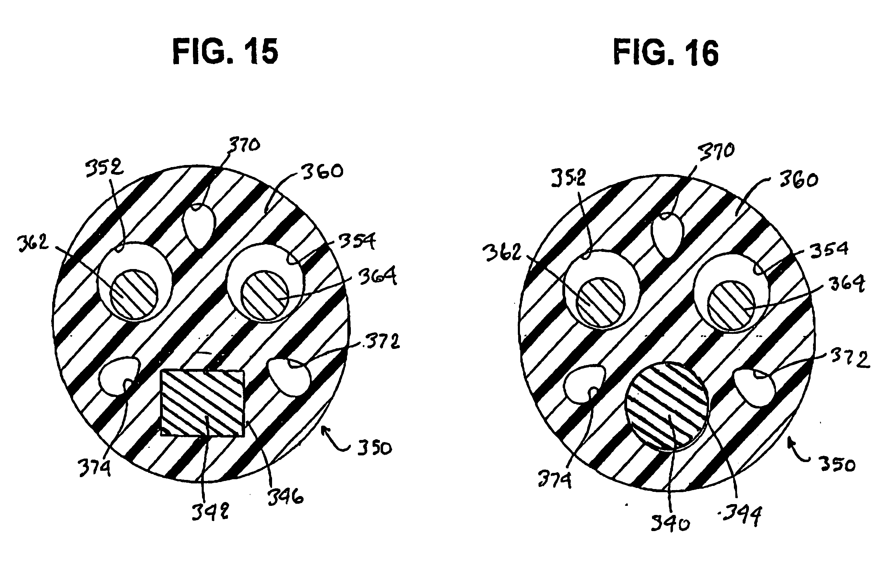

Preferred forms of the lead body 350 are depicted schematically in FIGs. 15 and 16, which is formed of straight, stranded wire conductors 362 and 364 disposed in separate parallel lumens 352 and 354 formed in the extruded insulator body 360. A circular or rectangular cross-section malleable member 340 or 342 is co-extruded with the insulator body 350 or inserted into malleable member lumens 344 and 346, respectively. Three compression lumens 370, 372 and 374 are also preferably extruded in the insulator body to relieve bending pressures that may be imposed by bends induced during introduction or within the heart chamber of coronary vessel as disclosed in commonly assigned U.S. Patent No. 5,584,873. FIGS. 15 and 16 merely illustrate one exemplary way in which the lead conductors and catheter body 350 can be fabricated in which the present invention can be advantageously employed.

The malleable member 340 or 342 can extend through the distal section 312 or a segment thereof and proximally to the junction 316 or all the way to the proximal connector 380. Preferably, the malleable member distal end is locked to the distal tip 318, and the malleable member proximal end is either locked to the connector 380 or to a hard plastic member formed of a relatively rigid PEEK or other hard material at or near the junction 316 to lessen the tendency of the malleable member to twist or rotate when a bend is formed in it. The co-extrusion of the malleable member with the catheter body 350 also lessens the tendency of the malleable member to twist or rotated as the distal section or a segment thereof is manipulated to form a shape of the types illustrated in FIGS. 2A-2H, for example, or as illustrated in the above-referenced '873 patent.

The malleable member 340, 342 is formed of a Beta III titanium alloy of the type exhibiting superelastic properties when subjected to strain at a bending strain of less than a set threshold strain and capable of undergoing plastic flow to take a set shape when subjected to a strain of greater than the set threshold at the same temperature, that is room temperature in this case. In accordance with the invention, the set shape is reversible so that any manually formed set shape of the malleable member that does not work in enabling conformance of the distal section to the anatomical structure of interest can be reversed. Moreover, the Beta III titanium alloy enables the set shape of the malleable member 340, 342 to be restored to substantially the imparted set shape following application of strain of less than the set threshold strain during introduction of the medical electrical lead 310 into a patient's body.

### Conclusion:

The malleable members illustrated in the drawings and described above as being circular or rectangular in cross-section are merely illustrative. Other cross-sections, e.g. hex-shaped, star-shaped or I-beam-shaped malleable members could also be advantageously employed, particularly when co-extruded into a polymeric tubular member to minimize the tendency to slip-rotated or twist axially when subjected to strain.

## Claims

1. An elongated medical device (10, 110, 210, 310) for introduction into a patient's body into conformance with an anatomical structure at a site of interest, comprising an elongated device body (50, 150, 350) having device body proximal (20, 120, 220, 320) and distal ends (18, 118, 218, 318) and a device body axis, the device body further comprising a device body proximal section (14, 114, 214, 314) extending from the device body proximal end to a proximal section distal end and a device body distal section (12, 112, 212, 312) extending from the proximal section distal end to the device body distal end; and a malleable member (40, 42, 140., 142, 340, 342) extending within the device body (50, 150, 350) along the device body axis, **characterised in that** the malleable member is formed of a Beta III titanium alloy of the type exhibiting superelastic properties when subjected to strain at a bending strain of less than a set threshold strain and capable of undergoing plastic flow to be manually formed in a first set shape of a plurality of set shapes when subjected to a strain of greater than the set threshold to impart a shape enabling conformance of the distal section to the anatomical structure, the Beta III titanium alloy enabling the malleable member to be formed in a second set shape different from the first set shape during application of strain of less than the set threshold strain during introduction of the elongated medical device into a patient's body and orientation of the distal segment into conformance with the anatomical structure at the site of interest.

2. The elongated medical device of claim 1, **characterized in that** the malleable member (40, 42, 140, 142, 340, 342) extends from the device body proximal end (20, 120, 223, 320) to the device body distal end (18, 118, 218, 318).

3. The elongated medical device of claim 1, **characterized in that** the malleable member (40, 42, 140, 142, 340, 342) extends through a segment of the distal section (12, 112, 212, 312).

4. The medical device of any preceding claim, the device being an elongated catheter having catheter body proximal and distal ends (118), a tubular side wall and a lumen (146) extending from lumen proximal and distal openings, and a catheter body axis.

5. The device of claim 4, further **characterized by** a manipulator (52) coupled between the catheter body proximal end and the catheter body distal section that enables deflection of the catheter body distal section from outside the body to facilitate introduction and orientation of the distal segment into conformance with the anatomical structure.

6. The elongated device as claimed in any preceding claim, the device being an electro-physiology catheter (10) for introduction into a heart chamber of a patient's heart into conformance with an anatomical structure of the heart wall at a site of interest to effect mapping and/or ablation of myocardial tissue, having
an elongated catheter body (50) having proximal (20) and distal (18) catheter body ends and a catheter body axis;
a handle (22) coupled to the catheter body proximal end (20) ;
a catheter body proximal section (14) joined at a proximal section distal end with a catheter body distal section (12);
an electrode (30, 28, 26, 24) formed in the distal section (12) for sensing cardiac signals during mapping and for delivering ablation energy during ablation;
an electrical conductor (62, 64, 66, 68) extending through the catheter body (50) from the electrode (30, 28, 26, 24) to the handle (22).

7. An elongated device as claimed in any of claims 1 to 4, the device being a guide wire (210) having an elongated guide wire body having guide wire body proximal (220) and distal ends (218) and a guide wire body axis, the guide wire body further comprising a guide wire body proximal section (214) extending from the guide wire body proximal end (220) to a proximal section (214) distal end and a guide wire body distal section extending from the proximal section distal end to the guide wire body distal end (218).

## Patentansprüche

1. Langgestreckte medizinische Vorrichtung (10, 110, 210, 310) zum Einführen in den Körper eines Patienten in Übereinstimmung mit einer anatomischen Struktur an einer Stelle von Interesse, mit einem langgestreckten Vorrichtungskörper (50, 150, 350) mit proximalen (20, 120, 220, 320) und distalen (18, 118, 218, 318) Enden des Vorrichtungskörpers und einer Vorrichtungskörperachse, wobei der Vorrichtungskörper ferner einen proximalen Abschnitt (14, 114, 214, 314) des Vorrichtungskörpers, der sich vom proximalen Ende des Vorrichtungskörpers zu einem distalen Ende des proximalen Abschnitts erstreckt, und einen distalen Abschnitt (12, 112, 212, 312) des Vorrichtungskörpers, der sich vom distalen Ende des proximalen Abschnitts zum distalen Ende des Vorrichtungskörpers erstreckt, aufweist; und einem verformbaren Element (40, 42, 140, 142, 340, 342), das sich innerhalb des Vorrichtungskörpers (50, 150, 350) entlang der Vorrichtungskörperachse erstreckt, **dadurch gekennzeichnet, dass** das verformbare Element aus einer Beta-III-Titan-Legierung der Art, die superelastische Eigenschaften zeigt, wenn sie einer Spannung mit einer Biegespannung von weniger als einer bestimmten Grenzwertspannung unterworfen ist, und die in der Lage ist, einen plastischen Fluss bzw. eine plastische Bewegung zu ertragen, um manuell in einer ersten bestimmten Form einer Anzahl von bestimmten Formen geformt zu werden, wenn sie einer Spannung größer als der bestimmte Schwellwert unterworfen ist, um eine Form zu verleihen bzw. bereitzustellen, die eine Übereinstimmung des distalen Abschnitts mit der anatomischen Struktur ermöglicht, wobei die Beta-III-Titan-Legierung ermöglicht, dass das verformbare Element in eine zweite bestimmte Form, die unterschiedlich zu der ersten bestimmten Form ist, während der Anwendung einer Spannung kleiner als die bestimmte Schwellwertspannung während der Einführung der langgestreckten medizinischen Vorrichtung in den Körper eines Patienten und der Orientierung des distalen Segments bzw. Bereiches in Übereinstimmung mit der anatomischen Struktur an der Stelle von Interesse geformt wird.

2. Langgestreckte medizinische Vorrichtung nach Anspruch 1, **dadurch gekennzeichnet, dass** sich das verformbare Element (40, 42, 140, 142, 340, 342) vom proximalen Ende (20, 120, 223, 320) des Vorrichtungskörpers zum distalen Ende (18, 118, 218, 318) des Vorrichtungskörpers erstreckt.

3. Langgestreckte medizinische Vorrichtung nach Anspruch 1, **dadurch gekennzeichnet, dass** sich das verformbare Element (40, 42, 140,142, 340, 342) durch ein Segment des distalen Abschnitts (12, 112, 212, 312) erstreckt.

4. Medizinische Vorrichtung nach einem der vorstehenden Ansprüche, wobei die Vorrichtung ein langgestreckter Katheter mit proximalen und distalen Enden (118) des Katheterkörpers, einer röhrenförmigen Seitenwand und einem Lumen (146), das sich von proximalen und distalen Lumenöffnungen erstreckt, und einer Katheterkörperachse ist.

5. Vorrichtung nach Anspruch 4, ferner **gekennzeichnet durch** einen Manipulator (52), der das proximale Ende des Katheterkörpers und den distalen Abschnitt des Katheterkörpers verbindet, was die Ablenkung bzw. Biegung des distalen Abschnitts des Katheterkörpers von der Außenseite des Körpers ermöglicht, um die Einführung und die Orientierung des distalen Segments in Übereinstimmung mit der anatomischen Struktur zu erleichtern.

6. Langgestreckte Vorrichtung nach einem der vorstehenden Ansprüche, wobei die Vorrichtung ein elektrophysiologischer Katheter (10) zum Einführen in eine Herzkammer eines Patientenherzes in Übereinstimmung mit einer anatomischen Struktur der Herzwand an einer Stelle von Interesse ist, um eine Kartierung und/oder Abtragung von Myokardgewebe zu bewirken, mit
einem langgestreckten Katheterkörper (50) mit proximalen (20) und distalen (18) Enden des Katheterkörpers und einer Katheterkörperachse;
einem Griff (22), der mit dem proximalen Ende (20) des Katheterkörpers verbunden ist;
einem proximalen Abschnitt (14) des Katheterkörpers, der an einem distalen Ende des proximalen Abschnitts mit einem distalen Abschnitt (12) des Katheterkörpers verbunden ist;
einer Elektrode (30, 28, 26, 24), die in dem distalen Abschnitt (12) gebildet ist, zum Erfassen von kardialen Signalen bzw. Herzsignalen während der Kartierung und zum Bereitstellen von Abtragungsenergie während der Abtragung;
einem elektrischen Leiter (62, 64, 66, 68), der sich durch den Katheterkörper (50) von der Elektrode (30, 28, 26, 24) zum dem Griff (22) erstreckt.

7. Langgestreckte Vorrichtung nach einem der Ansprüche 1 bis 4, wobei die Vorrichtung ein Führungsdraht (210) mit einem langgestreckten Führungsdrahtkörper mit proximalen (220) und distalen (218) Enden des Führungsdrahtkörpers und einer Führungsdrahtkörperachse ist, wobei der Führungsdrahtkörper ferner einen proximalen Abschnitt (214) des Führungsdrahtkörpers, der sich vom proximalen Ende (220) des Führungsdrahtkörpers zu einem distalen Ende des proximalen Abschnitts (214) erstreckt, und einen distalen Abschnitt des Führungsdrahtkörpers, der sich vom distalen Ende des proximalen Abschnitts zum distalen Ende (218) des Führungsdrahtkörpers erstreckt, aufweist.

## Revendications

1. Dispositif médical allongé (10, 110, 210, 310) destiné à être introduit dans le corps d'un patient en conformité avec une structure anatomique dans un site d'intérêt, comportant un corps de dispositif allongé (50, 150, 350) ayant des extrémités proximales (20, 120, 220, 320) et distales (18, 118, 218, 318) de corps de dispositif et un axe de corps de dispositif, le corps de dispositif comportant de plus une section proximale de corps de dispositif (14, 114, 214, 314) s'étendant de l'extrémité proximale de corps de dispositif jusqu'à une extrémité distale de section proximale et une section distale de corps de dispositif (12, 112, 212, 312) s'étendant de l'extrémité distale de section proximale à l'extrémité distale de corps de dispositif ; et un élément malléable (40, 42, 140, 142, 340, 342) s'étendant à l'intérieur du corps de dispositif (50, 150, 350) le long de l'axe de corps de dispositif, **caractérisé en ce que** l'élément malléable est formé d'un alliage de titane Bêta III du type affichant des propriétés superélastiques lorsque soumis à une contrainte à une contrainte de flexion inférieure à une contrainte de seuil établi et capable de subir un écoulement plastique pour être manuellement formé dans une première forme définie parmi une pluralité de formes définies lorsque soumis à une contrainte supérieure au seuil défini pour communiquer une forme permettant la conformité de la section distale à la structure anatomique, l'alliage de titane Bêta III permettant à l'élément malléable d'être formé dans une seconde forme définie différente de la première forme définie durant l'application de la déformation inférieure à la déformation de seuil défini durant l'introduction du dispositif médical allongé dans le corps du patient et l'orientation du segment distal en conformité avec la structure anatomique au site d'intérêt.

2. Dispositif médical allongé selon la revendication 1, **caractérisé en ce que** l'élément malléable (40, 42, 140, 142, 340, 342) s'étend de l'extrémité proximale de corps de dispositif (20, 120, 220, 320) jusqu'à l'extrémité distale de corps de dispositif (18, 118, 218, 318).

3. Dispositif médical allongé selon la revendication 1, **caractérisé en ce que** l'élément malléable (40, 42, 140, 142, 340, 342) s'étend à travers un segment de la section distale (12, 112, 212, 312).

4. Dispositif médical selon l'une quelconque des revendications précédentes, le dispositif étant un cathéter allongé ayant des extrémités proximale et distale de corps de cathéter (118), une paroi latérale tubulaire et une lumière (146) s'étendant à partir d'ouvertures proximale et distale de lumière, et un axe de corps de cathéter.

5. Dispositif selon la revendication 4, **caractérisé de plus par** un manipulateur (52) couplé entre l'extrémité proximale de corps de cathéter et la section distale de corps de cathéter qui permet la déviation de la section distale de corps de cathéter à partir de l'extérieur du corps pour faciliter l'introduction et l'orientation du segment distal en conformité avec la structure anatomique.

6. Dispositif allongé selon l'une quelconque des revendications précédentes, le dispositif étant un cathéter électro-physiologique (10) destiné à être introduit dans une chambre cardiaque du coeur d'un patient en conformité avec une structure anatomique de la paroi du coeur au niveau d'un site d'intérêt pour effectuer un mappage et/ou une ablation de tissu myocardique, ayant
un corps de cathéter allongé (50) ayant des extrémités de corps de cathéter proximale (20) et distale (18) et un axe de corps de cathéter,
une poignée (22) couplée à l'extrémité proximale de corps de cathéter (20),
une section proximale de corps de cathéter (14) raccordée au niveau d'une extrémité distale de section proximale à une section distale de corps de cathéter (12),
une électrode (30, 28, 26, 24) formée dans la section distale (12) pour détecter des signaux cardiaques durant le mappage et pour délivrer une énergie d'ablation durant l'ablation,
un conducteur électrique (62, 64, 66, 68) s'étendant à travers le corps de cathéter (50) de l'électrode (30, 28, 26, 24) à la poignée (22).

7. Dispositif allongé selon l'une quelconque des revendications 1 à 4, le dispositif étant un fil guide (210) ayant un corps de fil guide allongé ayant des extrémités proximale (220) et distale (218) de corps de fil guide et un axe de corps de fil guide, le corps de fil guide comportant de plus une section proximale de corps de fil guide (214) s'étendant de l'extrémité proximale de corps de fil guide (220) jusqu'à une extrémité distale (214) de section proximale et une section distale de corps de fil guide s'étendant de l'extrémité distale de section proximale jusqu'à l'extrémité distale de corps de fil guide (218).
